# EUROPEAN PATENT APPLICATION

(11) **EP 1 138 392 A2**
(43) Date of publication of application: **04.10.2001**
(21) Application number: 01107607.2
(22) Date of filing: 27.03.2001
(51) Int. Cl.: B04B 5/04, B04B 7/08

(54) **Centrifuge bowl for separating particles**

(30) Priority: 30.03.2000 JP 2000094690
(71) Applicant: Haemonetics Corporation, Braintree, Massachusetts 02184-9114 (US)
(72) Inventor: Sakota, Koichiro, Chuo-city Tokyo 1820014 (JP)
(74) Representative: Rupprecht, Kay, Dipl.-Ing.

(57) **Abstract**

A novel centrifuge bowl for processing particles suspended in a fluid is disclosed. The centrifuge bowl 10 includes an annular cavity 20 concentrically located about the rotation axis for suitably separating particles of similar densities but of different diameters. The cavity is preferably configured to have an annular cross sectional area, which is parallel to the rotation axis, that increases from a centrifugal side of the cavity toward a centripetal side of the cavity. This configuration allows to generate an almost rigidly rotating field upon rotation of the centrifuge bowl, which field helps to uniformly disperse Coriolis force throughout the circumference of the cavity to avoid turbulent mixing of the particles. In an alternative embodiment, the cavity is surrounded by an outer cavity for separating particles according to density before processing them through the inner cavity. This construction is particularly suitable for processing whole blood to harvest platelet-rich-plasma with reduced level of white blood cell contamination.

## Description

The present invention relates to the field of separating particles, in particular to a centrifuge bowl for separating particles of differing size and/or density suspended in a fluid. More specifically, when applied to the medical field, the present invention relates to an improved centrifuge bowl which enables to produce a blood product with a substantially lower level of contamination with white blood cells.

### BACKGROUND ART

In many fields of technology, it is desired to separate particles suspended in a fluid. For example, in the medical field, it is desired to fractionate whole human blood for transfusion purposes. Specifically, whole human blood includes blood cells such as red blood cells, white blood cells and platelets and these cells are suspended in plasma, an aqueous solution of proteins and other chemicals. Today, blood transfusions are widely given by transfusing only those blood components required by a particular patient instead of using a transfusion of whole blood. Transfusing only those blood components necessary saves the available supply of blood, and in many cases, is convenient for the patient.

To this end, whole human blood is separated into its various blood components with a procedure called apheresis. According to a typical apheresis process, whole blood is separated into a higher density component such as red blood cells, at least one intermediate density component such as platelets and white blood cells, including lymphocytes and granulocytes, and a lower density component such as plasma, and a desired blood component or components are harvested. Among various blood component products or fractionates obtainable through apheresis, the demand for concentrated platelet products is rapidly growing. This is particularly because, with the improvement in cancer therapy, there is a need to administrate more and more platelets to patients whose hemopoietic function is often lowered after undergoing chemical or radiation therapy.

As is well known, platelets have a short half-life of 4-6 days and the number of donors is usually limited. Therefore, in the production of concentrated platelet products, i.e., platelet-rich-plasma, it is important to harvest platelets from the whole blood supplied by a donor at a maximum yield. Further, it is known that contamination of concentrated platelet products with white blood cells can lead to serious medial complications, such as GVH reactions. Therefore, it is also very important to keep the level of contamination with white blood cells as low as possible, while maximizing platelet yields.

Recent immunology studies have revealed that side effects of contaminant white blood cells can be substantially reduced, if not completely obviated, when the level of contamination is sufficiently low. For example, it has been reported that non-hemolytic febrile reaction (NHFR) rarely takes place if the number of white blood cells contained in a 200 ml transfusion bag, which may contain 2.0-3.0 × 10⁻¹¹ platelets, is 5.0 × 10⁷ or less. Likewise, it has been recognized that complications such as CMV viral infection and alloimmunization rarely take place if the level of white blood cells in a bag is 1.0 × 10⁷ and 1.0 × 10⁶, respectively, or lower (Kazuo Tsubaki, *Saishin Igaku* Vol. 48, No. 7, pp. 989-996 (1993)). In view of these, it is desired to consistently produce, through apheresis, concentrated platelet products having 1.0 × 10⁶ or less white blood cells.

Among several apheresis methods available, an intermittent flow method and a continuous flow method have been widely used. Further, centrifugation has been widely accepted as a technique for separating blood components according to density or specific gravity. A centrifuge bowl of the type disclosed in U.S. Patent No. 4,300,717, herein referred to as "Latham" bowl, typifies a centrifuge bowl for use in the intermittent flow method. The bowl comprises a rotor portion in which blood components are separated and a stator portion having inlet and outlet ports, and these are combined by a rotary seal. The rotor portion comprises a generally frustoconical body and a similarly shaped core is coaxially disposed therein to form a fractionation chamber therebetween. In use, anticoagulated whole blood is introduced to the bowl through the inlet port. The rotor rotates at a fixed or variable speed and blood components are separated within the fractionation chamber by centrifugation in accordance with density. With blood continuously entering the bowl through the inlet port, the separated blood components are progressively displaced inwardly from the radially outward portion of the bowl and successively reach the outlet port. Blood components exiting through the outlet port are retained and stored, while components remaining in the bowl are usually returned to the patient or donor.

To maximize the yield of platelets while decreasing white blood cell contaminants, various excellent techniques have been developed in connection with the Latham bowl. For example, in Schoendorfer et al. U.S. Patents No. 4,416,654 and No. 4,416,654 assigned to the same assignee of the present application, a "surge" technique is disclosed. According to the surge technique, when whole blood is collected and separated within the fractionation chamber into a red blood cell layer, a buffy coat layer which is a mixture of platelets and white blood cells and a plasma layer, a low density fluid, preferably plasma, is pumped through the centrifuge at a relatively high flow rate. The platelets and white blood cells in the buffy coat layer, which are of similar densities but of different effective diameters, are centrifugally elutriated and the yield of platelets is improved thereby.

Further, according to Latham et al. U.S. Patent No. 5,607,579 also assigned to the assignee hereof, the separation between platelets and white blood cells is further improved by stopping withdrawal of whole blood and recirculating plasma through the centrifuge prior to the surge phase. This technique is called "dwell", during which platelets and white blood cells are effectively separated and arranged in the order of size, before being displaced from the centrifuge using the surge technology. The '579 patent also teaches to recirculate plasma while the withdrawal of whole blood so as to dilute the same and to promote separation among the blood components.

In accordance with the dwell technology, the level of contamination with white blood cells is decreased to the order of 1.0 × 10⁷ per transfusion bag containing platelets at the usually required dosage. To meet the demanding therapeutic needs of today, however, it is desirable to further decrease the level of contamination.

In the field of continuous flow apheresis method, on the other hand, centrifugal elutriation has also been widely employed. For example, U.S. Patents No. 4,268,393; No. 4,269,718; No. 4,350,283 and No. 4,798,579 describe a funnel-shaped or cone-shaped chamber rotatable with a centrifuge around a rotation axis for performing centrifugal elutriation. Generally, the chamber diverges from an inlet disposed at a centrifugal side toward an outlet disposed at a centripetal side. As a low density fluid, such as plasma, is pumped through the chamber, smaller cells having a slower sedimentation velocity are allowed to exit from the chamber through the outlet, while larger cells having a faster sedimentation velocity are retained within the chamber. By appropriately controlling the speed of rotation, cells having a desired diameter can be successively elutriated from the chamber.

However, the centrifugal elutriation with this type of chamber suffers from a number of inherent disadvantages. Specifically, when cells enter the chamber rotating around the centrifuge axis and plasma is pumped through the chamber, Coriolis force which is known to give rise to a whirling flow is generated and the cells and the plasma turbulently flow along the chamber wall facing the direction of rotation of the centrifuge. This mixes the cells being separated within the chamber, and also directly routes the cells from the inlet to the outlet without passing through the region where the centrifugal elutriation theoretically should take place. This reduces the effectiveness of the centrifugal elutriation considerably. Another problem with the prior art centrifugal elutriation is cell mixing by density inversion. As the chamber is diverging from the inlet to the outlet, the velocity of the cells entering the chamber decreases as they move from the inlet to the outlet. This leads to a high concentration near the outlet and a low concentration near the inlet. This condition is unstable and may lead to turnover and turbulent mixing when the centrifugal force urges the cells in the high concentration region near the outlet toward the inlet region.

Hlavinka et al. U.S. Patent No. 5,674,173 describes a technique for mitigating the aforementioned problems while benefiting from centrifugal elutriation. According to the '173 patent, a chamber having a kite-shaped axial cross section is mounted on a centrifuge for rotation therewith. The interior of the chamber converges from a maximum cross-sectional area near an outlet toward an inlet. The interior includes one or more grooves surrounding the longitudinal axis of the chamber for dispersing Coriolis force in a circumferential direction around the longitudinal axis. However, the shape of the chamber of the '173 patent is still generally conical and Coriolis force may not be sufficiently dispersed through the grooves and may still cause turbulent mixing of the separated cells along the chamber wall facing rotation. Further, while the '173 patent describes that a saturated bed of platelets is established at the maximum cross-sectional area and this bed rejects white blood cells, circular current could be formed between the platelet bed and upstream plasma and this may also cause whirl mixing of the cells.

### OBJECT OF THE INVENTION

Accordingly, an object of the present invention is to provide an improved centrifuge bowl for separating particles suspended in a fluid, in particular blood components or cells of whole blood.

Another object of the present invention is to provide a centrifuge bowl for separating or harvesting platelets at a high yield, with a sufficiently low level of contamination with white blood cells.

A further object of the present invention is to provide a centrifuge bowl which can be mounted to a conventional apheresis machine and can be operated in accordance with conventional protocols, while providing the aforementioned advantages.

A further object of the present invention is to provide a centrifuge bowl which is simple in structure and less costly in manufacture.

### DISCLOSURE OF THE INVENTION

According to one aspect of the present invention, a centrifuge bowl comprises an inlet port, an outlet port and at least one annular cavity concentrically located about the rotation axis of the centrifuge bowl. The annular cavity communicates with the inlet and outlet ports at its centripetal and centrifugal peripheries, respectively, so that fluid entering the inlet port flows through the cavity toward the rotation axis before exiting the outlet port.

Preferably, the bowl comprises a hollow bowl body having an aperture at one axial end and the inlet and outlet ports fluidly communicate with the interior of the bowl body through the aperture, for example by way of tubing. A rotary seal is disposed to cover the aperture if needed. A core may be disposed within the hollow interior of the bowl body for rotation therewith, and the annular cavity is defined between the bowl body and the core. The bowl body and the core may also form an axial gap therebetween to define a passageway for directing fluid from the inlet port radially outwardly to the centrifugal periphery of the cavity.

This type of centrifuge bowl is suitable for processing a fluid containing first particles and second particles, which are of similar densities but of different diameters. A fractionated whole blood containing platelets and white blood cells suspended in plasma is a good example of such fluid. The bowl may be employed, for example, as a secondary centrifuge in an apheresis machine and operable for receiving from a primary centrifuge platelet-rich-plasma and purifying the same by decreasing the level of contamination with white blood cells. However, other uses may be readily apparent to those skilled in the art and they are within the scope of the present invention. For example, other blood fractions may be suitably processed through the bowl. Also, the bowl may be appropriately scaled and configured to process whole blood rather than blood fractions. Further, it is also within the skill of an artisan to provide, where necessary, two or more such annular cavities in succession.

Principally, it is believed that particles suspended in a fluid are separated by centrifugal elutriation as the fluid flows through the annular cavity while the centrifuge bowl is rotating. Specifically, in the case of plasma containing platelets and white blood cells, the particles are of similar densities. As the white blood cells have a greater diameter, however, they have a faster sedimentation velocity than the platelets, according to Stoke's law. Therefore, by pumping such plasma suspension from the inlet port to the outlet port through the cavity, the platelets are collected at a high yield while the white blood cells remain within the cavity. From this point of view, the present invention may have something in common with the prior art centrifugal elutriators discussed above.

However, the cavity in accordance with the present invention is annular in shape, while the chambers of the prior art centrifugal elutriators are not. As mentioned previously, the prior art centrifugal elutriators suffer from adversarial effects of Coriolis force which causes turbulent mixing of separated particles along the chamber wall facing rotation. In accordance with the present invention, it is believed that Coriolis force is not localized and uniformly dispersed around the cavity because of the annular configuration of the cavity. Therefore, the prior art problems associated with localization of Coriolis force can be obviated by the present invention.

Further, while the inventors do not wish to be bound by a particular theory or function, it is also believed that a phenomenon known as almost rigidly rotating flow is created within the annular cavity. Specifically, when a fluid containing suspended particles is introduced into and fills the cavity while the centrifuge bowl is rotating, flow of the fluid mostly takes place through thin layers known as Stewartson and Ekman layers formed along cavity walls. These layers are considered to be established rapidly when the centrifuge bowl is accelerated sufficiently, and disperse the angular momentum of the system throughout the cavity. Because of this, a turbulent flow is not generated within the cavity. The particles are separated by centrifugal elutriation as they move through the Stewartson and Ekman layers, and particles having a faster sedimentation velocity are either prevented from exiting the cavity or deviated out of these layers and taken into an interior flow region formed between the layers. It is also speculated that a portion of the particles somehow circulate with fluid within the cavity and this promotes separation between the particles.

The annular cavity may assume various different configurations within the frame of the present invention. One suitable configuration of the annular cavity is such that its annular cross sectional area, which is taken along an imaginary cylinder extending in a direction parallel to the rotation axis, increases as the diameter of the imaginary cylinder decreases. This means that the value described as 2πrh, wherein r is a radial distance from the rotation axis and h is the height of the cavity parallel to the rotation axis at that radial distance, increases as the value of r decreases. If the annular cavity suffices this condition, the flow velocity through the cavity decreases as the fluid entering the cavity at the centrifugal periphery thereof, i.e., radially outer side of the annular cavity, flows toward the centripetal periphery, i.e., radially inner side of the annular cavity. This is usually preferable for separating particles suspended in a fluid by centrifugal elutriation. As an example, the annular cavity may have a triangular or quadrangular shape in axial cross section, but it should be understood that other geometrical shapes are also possible. Preferably, the maximum annular cross sectional area is located near the cavity outlet, but this does not preclude to provide an annular transition area, in which the annular cross sectional area decreases from the maximum annular cross sectional area toward the cavity outlet.

Further, it may be preferable if the centrifugal periphery of the annular cavity, which is in fluid communication with the inlet port of the centrifuge bowl and defining a peripheral slot for fluid entry into the cavity, is vertically offset along the rotation axis from the centripetal periphery of the annular cavity, which is in fluid communication with the outlet port of the centrifuge bowl and defining a peripheral slot for fluid exit from the cavity. This configuration may prevent fluid flow from radially directly routed from the centrifugal periphery to the centripetal periphery before the particles are sufficiently separated through the cavity. More preferably, the axial cross section of the cavity is asymmetric with respect to a line drawn to pass through the cavity inlet and outlet. It is also preferable that the cavity terminates at its centripetal side with a cylindrical wall extending along the rotation axis and the peripheral cavity outlet is formed intermediate between the upper and lower peripheral edges of the cylindrical wall.

In accordance with another aspect of the present invention, a centrifuge bowl having a radially outer cavity and a radially inner cavity is provided. This is particularly suitable for processing a liquid including particles of different densities, as well as particles of similar densities but of different diameters. Whole blood is a typical example that is amenable to processing with this bowl. With this type of centrifuge bowl, it is possible to advantageously replace a conventional centrifuge bowl, such as the Latham bowl, and an improved separation can thereby be achieved without necessitating substantial modification to the existing apheresis machines that utilize the Latham bowl.

Preferably, the centrifuge bowl in accordance with this aspect comprises a bowl body concentrically located about the rotation axis and a core disposed within the bowl body for rotation therewith, and the outer and inner annular cavities are defined therebetween. The outer cavity includes a centrifugal peripheral slot which is in fluid communication with an inlet port and the inner cavity includes a centripetal peripheral slot which is in fluid communication with an outlet port. The inner and outer annular cavities fluidly communicate with each other through an annular restriction channel formed between the cavities. Preferably, the annular restriction channel communicates the cavities solely in the radial direction so that the overall height of the centrifuge bowl can be decreased. The annular channel has a short axial height and flow from the outer cavity is throttled through the annular channel. Generally, the inner cavity is constructed to perform the same function as the annular cavity previously discussed, and thus the description regarding the annular cavity set forth above may equally be applied to the inner cavity. The outer cavity usually serves to separate particles according to density, and its configuration may be limited only from this point of view. To enable easy manufacture, the outer cavity is preferably formed to have a simple shape, for example a rectangular shape in axial cross section.

The bowl body may include an aperture at one axial end thereof and a rotary seal assembly which includes the inlet and outlet ports may be affixed to the bowl body to cover the aperture. The bowl body may have a two-part construction including a disc-like bottom wall and a shaped upper part which may be formed by injection molding. To assemble a bowl, the core is positioned on the bottom wall so as to define a radial gap or passageway between a lower surface of the core and an upper surface of the bottom wall, and the upper part is then placed over the core. The upper part is then integrated with the bottom wall by hermetically sealing them together at the periphery, and a rotary seal assembly is inserted through the axial aperture of the bowl body to cover the same. This type of centrifuge bowl is simple in structure and can be inexpensively manufactured.

The inlet port fluidly communicates with the radial passageway formed along the bottom wall of the bowl body, and a fluid pumped into the inlet port is directed radially outwardly through the passageway to enter the outer annular cavity at the centrifugal periphery thereof. The fluid then enters the annular inner cavity through the annular channel and exits from the outlet port which is in fluid communication with the annular inner cavity.

When whole blood is pumped through the inlet port and guided into the centrifuge bowl, it is led through the radial passageway and enter the outer annular cavity from the centrifugal peripheral slot. Within the outer annular cavity, the whole blood is separated by a centrifugal force and stratified in accordance with density. At this point, a layer of red blood cells, a buffy coat layer and a plasma layer are formed. With continued withdrawal of whole blood, the separated blood components enter, with the plasma layer first, into the inner cavity via the restricting annular channel. In the inner cavity, the components of the buffy coat layer are separated by centrifugal elutriation, and perhaps under the influence of an almost rigidly rotating flow, as described above.

Preferably, the centrifuge bowl in accordance with this aspect of the present invention is dimensioned to have the same diametrical size as the conventional Latham bowl, so that it can be mounted to a conventional apheresis machine such as MCS, Multi or CCS manufactured by Haemonetics Corporation of 400 Wood Road, Braintree, MA 02184, U.S.A., the assignee hereof. Further, the radial position of the cavities and annular channel is adjusted so that the bowl would be compatible with the existing optics and/or electronics of the conventional apheresis machines.

In accordance with a typical protocol for harvesting platelet-rich-plasma, anticoagulated whole blood is drawn into the bowl at a speed of 20 to 200 ml/min, preferably 50 to 150 ml/min. When the whole blood is separated within the outer cavity and the front end of the buffy coat layer has approached or entered the annular channel, blood drawing is stopped and plasma is recirculated at a surge flow rate, e.g., at a speed in the range of 120 to 240 ml/min for effectively separating platelets and white blood cells. By this process, platelets are selectively pumped out of the inner cavity through the outlet, while white blood cells are retained in the inner cavity. The remaining blood fraction in the centrifuge bowl is then returned to the patient or donor. Prior to the surge step, a dwell step may be performed, in which plasma is recirculated at a constant or gradually increasing speed within a range of 60 to 160 ml/min without causing platelets to egress from the outlet port. Further, during the drawing of whole blood into the bowl, it is possible to dilute or compensate for the flow by circulating plasma. This is known as the critical flow technology.

The foregoing and other objects, features and advantages of the present invention will be apparent from the following detailed description of preferred embodiments shown in the drawings. The drawings are not necessarily to scale, and emphasis might have been placed to illustrate the principles of the present invention.

### DESCRIPTION OF PREFERRED EMBODIMENTS

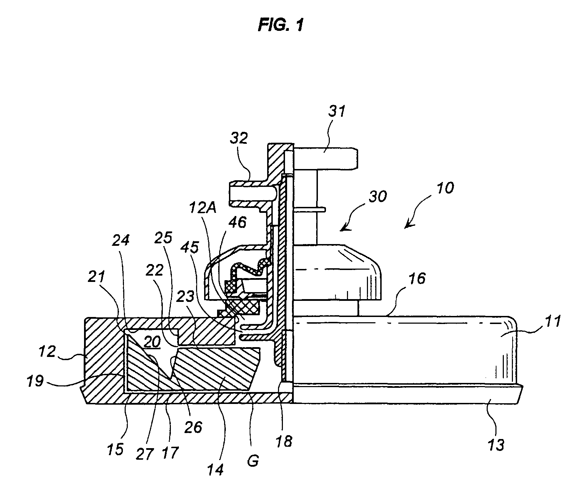

Referring now to Fig. 1, an embodiment of a centrifuge bowl 10 having a single annular cavity 20 is shown. In the following, the bowl will be described as a disposable centrifuge bowl adapted for the processing of a fractionated whole blood, e.g., platelets and white blood cells suspended in plasma (hereinafter "platelet-rich-plasma fraction", or "PRP fraction"), but it should be understood that the invention will not be limited thereto in any way. For example, the bowl of Fig. 1 may be employed, with or without modification, for processing other whole blood fractions or whole blood *per se*.

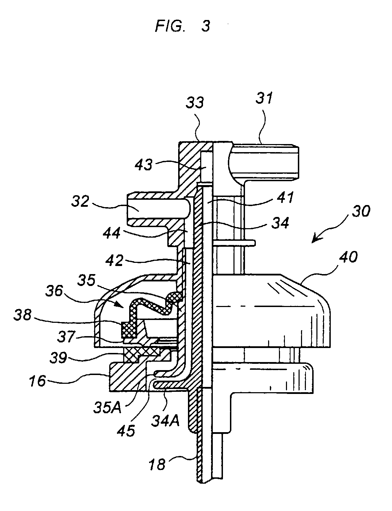

As shown in Fig. 1, the bowl comprises a rotary seal assembly, or seal and header assembly, shown generally at 30, a bowl body shown generally at 12 and a core 14. The seal and header assembly 30 is shown in more detail in Fig. 3. It provides a rotary seal and a fluid communication pathway between the interior of the rotatable bowl body 12 and stationary conduits 41 and 42 connected respectively to an inlet port 31 and an outlet port 32. The assembly 30 is comprised of a stationary header, shown generally at 33, a feed tube assembly, shown generally at 34, an effluent tube 35, and a rotary seal, shown generally at 36. The rotary seal 36 comprises a fixed seal ring 37, a diaphragm member 38 and a rotatable seal ring 39 which is disposed on an outside seal member, or crown 16. The diaphragm member 38 is affixed about its outer periphery to the periphery of the seal ring 37. The seal ring 37 includes an annular lip which slidably contacts against an opposing surface of the seal ring 39. The crown 16 may include an axially open groove on its periphery or may otherwise be appropriately configured to establish a fluid-tight coupling with the bowl body 12. The crown 16 is provided with a central opening, through which the effluent tube 35 extends. The inner periphery of the diaphragm member 38 is joined to the effluent tube 35.

The header 33 is comprised of an integrally formed member having the inlet port 31, extending radially into an axial passageway 43. The passageway 43 is coupled to the inner, stationary conduit 41 formed by an axially extending bore of the feed tube assembly 34 and, in turn, to a feed tube stem 18, thereby forming a non-rotating inlet path for a PRP fraction to enter the interior of the bowl body 12. The header 33 also includes the outlet port 32, which extends radially into a channel 44 extending about the feed tube assembly 34 in coaxial relationship. The channel 44 then couples to the stationary conduit 42 to form an outlet passageway. An outer shield member 40 is formed on the header 33 and extends over the rotary seal 36.

The feed tube assembly 34 is formed with a radial flange 34A integral therewith and a radial flange 35A is also integrally formed on the effluent tube 35, thereby forming a radially outwardly opening collection port 45 fluidly communicating with the outlet port 32. The header and seal assembly 30, as thus described, is formed and assembled as an individual unit and, after the core 14 has been disposed within the bowl body 12 as shown in Fig. 1, inserted through the opening 12A of bowl body 12 and fixed thereto by appropriate means such as welding or threading.

The bowl body 12 is of a two-part construction, comprising a molded upper part 11 having axial openings and a molded lower part or bottom disc 13. Theses parts are made of any suitable plastic materials such as acrylics or styrene, which are compatible with physiological fluid. After the core 14 has been positioned on the bottom disc 13, the upper mold part 11 and the disc 13 are assembled and hermetically sealed together, for example by ultrasonic welding. The disc 13 or the core 14 includes spacers 15 which are circumferentially disposed at predetermined intervals, say at every 60°, so that the disc 13 and the core 14 are separated by an axial gap G which serves as a radial passageway 17 for guiding the PRP fraction introduced from the feed tube stem 18 to the cavity 20.

The annular cavity 20 is formed between and bounded by the lower surface of the upper bowl part 11 and the upper surface of the core 14. The cavity 20 includes a cavity inlet 21, which is a peripheral slot formed at the centrifugal side thereof, and a cavity outlet 23, which is a peripheral slot formed at the centripetal side thereof. The inlet slot 21 communicates with the radial passageway 17 through an axially extending, circumferentially continuous slit 19 and the outlet slot 22 communicates with the collection port 45 through a radially extending passageway 23 formed between the upper bowl part 11 and the core 14.

The annular cavity 20 has a generally triangular shape in axial cross section. The height of the cavity 20 increases from the inlet slot 21 toward the outlet slot 22 so that the annular cross sectional area given by 2πrh, wherein r is a radial distance from the rotation axis and h is the height of the cavity 20 parallel to the rotation axis at that radial distance, increases as the value of r decreases. The cavity 20 has an upper annular wall 24 which horizontally extends radially inwardly from the inlet slot 21 toward the rotation axis of the centrifuge 10 and terminates at an upper peripheral wall 25 which extends downwardly therefrom to the upper peripheral edge of the outlet slot 22. From the lower peripheral edge of the outlet slot 22, a lower peripheral wall 26 begins and extends generally in parallel to the rotation axis until it meets an inclined wall 27 extending from the inlet slot 21 radially inwardly and axially downwardly. The inclined wall 27 contains an acute angle with the rotation axis. This angle may range from 20 to 60 degrees, preferably is in a range of 30 to 50 degrees. The annular wall 24 may be modified to contain an obtuse angle with the rotation axis. Further, while the inclined wall 27 and the annular wall 24 are illustrated as a radially extending planar surface, they can alternatively be formed as a convexly or concavely curved surface insofar as smooth transitions of fluid and/or particles along the wall surfaces are maintained.

In this configuration, the inlet slot 21 and the outlet slot 22 are axially offset from each other by a distance approximately equal to the axial length of the upper peripheral wall 25. The amount of this offset may arbitrary be determined and there may be cases where the offset becomes zero. Generally, however, it is preferred not to dispose the inlet and outlet slots 21 and 22 in a coplanar relationship so that a direct radial pathway between these slots will not be formed, as previously mentioned. It may be more preferable to shape the cavity 20 and arrange the inlet and outlet slots 21 and 22 in such a manner that, when viewed in axial cross section, the cavity is not symmetrical with respect to a line drawn through the slots 21 and 22.

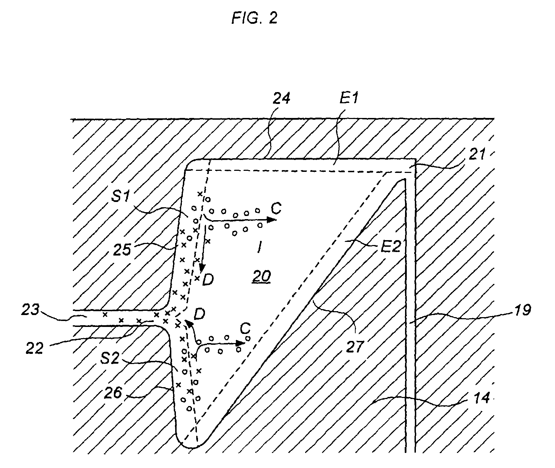

Referring now to Fig. 2, the expected function of the annular cavity 20 is described. When platelet-rich-plasma, or a PRP fraction is pumped into the bowl 10 through the inlet port 31, it flows through the conduit 41 and the stem 18 to the bottom portion of the bowl. The PRP fraction is led through the radial passageway 17 and circumferential slit 19 to enter the radial cavity 20 through the inlet slot 21.

With continued pumping of the PRP fraction into the bowl 10, the cavity 20 is filled therewith. Because the bowl 10 is rotated at a sufficient speed, for example at a speed of 2,000 to 7,000 rpm, preferably 3,000 to 5,000 rpm, a field of flow called an almost rigidly rotating flow is created within the annular cavity 20. The PRP fraction may be pumped into the bowl 10 at a flow rate of 20 to 200 ml/min, preferably 50 to 150 ml/min. The flow rate may substantially be constant over time. Alternatively, it may be increased over time or a combination of constant and increasing flow rates may be used.

Under the almost rigidly rotating flow field, Stewartson layers S1, S2 are created along the walls 25 and 26 and Ekman layers E1, E2 are created along the walls 24 and 27. Flow of the PRP fraction mostly takes place through these layers, and flow rarely takes place through an internal region indicated at I. It may be preferable that the outlet slot 22 opens at a position vertically intermediate the inner edges of the upper and lower annular walls 25 and 26 so that any particles moved along the Ekman layers E1, E2 will not directly travel into the outlet slot 22.

When platelets and white blood cells contained in the PRP fraction enter the cavity 20, they first move along the Ekman layers E1, E2 and then the Stewartson layers S1, S2, and are separated by centrifugal elutriation. In particular, when the PRP fraction flows through the Ekman layers E1 and E2, because platelets have a slower sedimentation velocity than white blood cells, the platelets are dragged by fluid flow more rapidly than the white blood cells toward the outlet slot 22. Part of the white blood cells, if not most, are retained in the Ekman layers because of their faster sedimentation velocity. In the Stewartson layers S1 and S2, while platelets (represented by "x") are subjected to an axial dragging force D created by the flow of viscous plasma and allowed to proceed to the outlet slot 22, larger white blood cells (represented by "o") are subjected to a centrifugal force C created by the rotation of the centrifuge 10 and taken into the internal region I. The inwardly diverging contour of the cavity 20 decreases the flow velocity of the PRP fraction as it moves inwardly toward the axial walls 25, 26. Because the cells in the PRP fraction are thus centrifugally elutriated through the Ekman and Stewartson layers, and perhaps because of the fact that turbulent flows due to Coriolis force are suppressed or not generated under the almost rigidly rotating flow field, good separation between platelets and white blood cells is considered to be achieved.

Platelets and plasma exits from the outlet slot 22 and guided through the radial passageway 23 and enter a collection chamber 46, in which the collection port 45 opens radially outwardly. It should be noted that, when the front end of the plasma displaced from the chamber 20 and the passageway 23 moves radially inwardly and reaches the collection port 45, air cannot escape from within the bowl and thus the collection chamber will not be overfilled with the plasma.

Typically, the diameter of the bowl 10 is 10-30 cm, depending upon the required processing volume. Preferably, it is within the range of 15-20 cm so that it can be mounted to a conventional apheresis machine without a substantial modification.

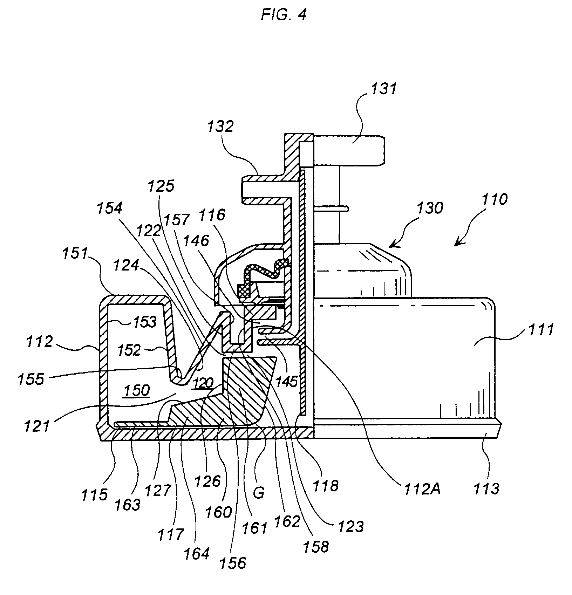

Turning now to Fig. 4, an embodiment of a centrifuge bowl 110 having an inner annular cavity 120 and an outer annular cavity 150 is shown. The centrifuge bowl 110 comprises a disposable centrifuge rotor, or bowl 112 which has an aperture 112A at one end, a rotary seal assembly 130 and a core 160. The rotary seal assembly 130 is substantially of the same construction as the rotary seal assembly 30 described above with reference to Fig. 3, and thus is not detailed herein.

As with the case of the bowl 10, the bowl body 112 may be formed of any suitable plastic material such as transparent styrene resin or the like and comprises an upper molded part 111 and a lower molded part or bottom disc 113. The crown 116 of the rotary seal assembly 130 is affixed to the aperture 112A by threading, welding or the like.

The core 160 disposed within the bowl body 112 has a stepped profile and comprises a hub 161 including a tapered bore 162, a radial disc 163 extending radially outwardly from one end of the hub 161, and an annular shoulder 164 contained between the hub 161 and the disc 163. Such a core can be easily manufactured by injection molding. Angularly separated spacers 115 are disposed between the bottom disc 113 of the bowl and the core 160 at, for example, intervals of 60°, thereby defining an axial gap G which serves as a radial passageway 117 for guiding fluid pumped into the bowl through the feed tube stem 118.

The upper molded part 111 is of a shape such that it cooperates with the stepped profile of the core 160 to define the inner and outer annular cavities 120, 150. Specifically, in the illustrated example, the upper molded part 111 comprises an outer cylindrical portion 151 having inner and outer walls 152, 153, a conical slope portion 154 and a neck portion 155 bridging between the inner wall 152 and the slope portion 154. With the radial disc 163 of the core 160, the outer cylindrical portion 151 defines the outer cavity 150 which is generally of an annular shape having a rectangular cross section. The outer cavity 150 communicates at its lower centrifugal periphery with the radial passageway 117. The neck and slope portions 155, 154 cooperate with the shoulder 164 of the core 160 and define the inner annular cavity 120. The outer and inner cavities 150, 130 communicate with each other through an annular channel 121 located between the uprising portion of the shoulder 164 and the neck portion 155.

As shown in Fig. 4, the inner cavity 120 is axially bounded between an upper annular wall 124, which is defined by the slope portion 154 extending radially inwardly and axially upwardly from the upper edge of the annular channel or inlet slot 121, and a lower annular wall 127 defined by the upper surface of the shoulder 164 which extends radially inwardly and axially upwardly from the lower edge of the annular channel 121. The upper wall 124 contains an acute angle with the rotation axis and the lower wall 127 contains an obtuse angle with the rotation axis. The upper annular wall 124 is therefore steeper than the lower annular wall 127 and thus the axial height of the inner cavity 120 increases as the distance from the rotation axis decreases. Preferably, the decrement of the radial distance is smaller than the increment of the axial height, Δr < Δh, so that the annular cross sectional area of the annular cavity 120 given by 2πrh increases as the value of r decreases. The upper and lower annular walls 124, 127 may be formed to have a curved profile, as in the case of the annular wall 24 and the inclined wall 27 described above in connection with Fig. 1.

The cavity 120 terminates at its radial inner end with upper and lower peripheral walls 125 and 126, between which a peripheral outlet slot 122 is defined. In this configuration, the inlet slot 121 and the outlet slot 122 are axially offset as in the embodiment of Fig. 1, but their relative axial positions are reversed.

The upper peripheral wall 125 is defined by an outer wall 157 of an inner cylindrical portion 156 and the lower peripheral wall 126 is defined by an outer peripheral wall of the hub 161. The inner cylindrical portion 156 is spaced from the upper surface of the hub 161 by a radial passageway 123 which communicates the inner cavity 120 with a collection chamber 146 defined between the inner wall 158 of the cylindrical portion 156 and the collection port 145.

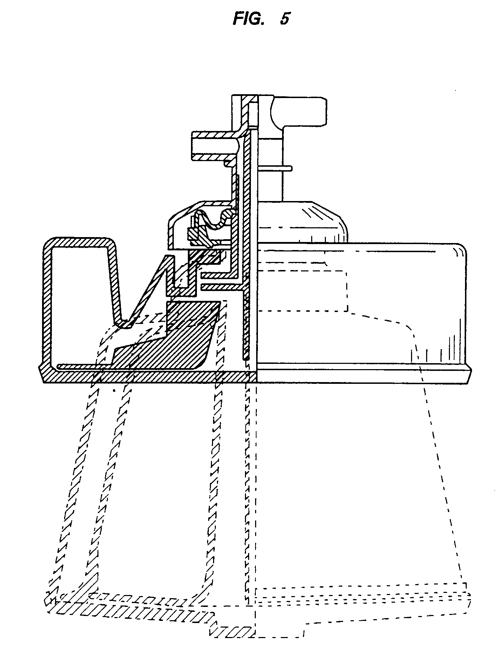

It is now in order to describe the operation of the centrifuge bowl of Fig. 4. The bowl 110 is particularly suitable for fractionating whole blood and harvesting platelets at a high yield. As shown in Fig. 5, the bowl of Fig. 4 may preferably be comparable with the conventional Latham bowl in diametrical size but has a reduced axial height. Therefore, by attaching an appropriately formed adapter or the like to compensate for the height, the bowl 110 can be mounted to a conventional apheresis machine such as MCS, Multi or CCS mentioned earlier, and operated with existing protocols using the existing optics and/or electronics. Of course, however, this does not exclude other bowl configurations. Broadly, the bowl 110 may have a diameter of 10-30 cm, preferably 15-20 cm.

First, with the use of a peristaltic pump (not shown), anticoagulated whole blood is drawn from a patient or donor and guided into the bowl 110 via the inlet port 131, and the bowl is started to rotate. The drawing of blood is usually made at a flow rate of 20 to 150 ml/min, preferably 50 to 100 ml/min, and the bowl may be rotated at a speed of 2,000 to 7,000 rpm, preferably 3,000 to 5,000 rpm. During the drawing of blood, the flow rate may substantially be constant over time. Alternatively, it may be continuously or stepwisely increased over time or a combination of constant and increasing flow rates may be used.

The whole blood is led from the inlet port 131 to the radial passageway 117 through the feed tube stem 118, and enter the outer annular cavity 150 at its lower peripheral edge. The whole blood is centrifugally separated and stratified into a layer of red blood cells, which is radially outermost within the outer cavity 150, and the inner, buffy coat and plasma layers. With continued withdrawal of whole blood, the separated blood components enter, with the plasma layer first, into the inner cavity 120. The fractionated blood components are then displaced from the inner cavity 120 through the radial passageway 123 and collected by the collection port 145. As in the case of the bowl of Fig. 1, when the front end of the displaced components reaches the collection port 145, air is trapped centrally of the bowl and thus the collection chamber 146 will not be overfilled with blood components. The fractionated plasma flows out from the outlet port 132 and collected in a storage bag (not shown).

When the front end of the buffy coat layer has approached the annular channel or inlet slot 121, a surge step may be started for separating platelets and white blood cells resident in the buffy coat layer. The surge can be started earlier or later, for example when the front end of the buffy coat layer has entered the inlet slot 121 or the inner cavity 120. The front end of the buffy coat, as well as other boundaries among the separated blood components can be detected, for example, with an optical sensor which monitors the radius of the region occupied by the separated blood component in the centrifuge and signals when the radius has reached a particular value.

To perform the surge, withdrawal of whole blood is stopped and part of the collected plasma is recirculated from the storage bag into the bowl 110 at an increased flow rate, for example a flow rate selected within a range of 120 to 240 ml/min, preferably 160 to 220 ml/min. The components in the buffy coat enter the inner cavity 120 and separated by centrifugal elutriation as they migrate through Ekman and Stewartson layers, as in the case of the cavity 20 shown and described in connection with Fig. 2. After most of the platelets have been collected in a storage bag, the plasma introduction is stopped. As can be recognized by those skilled in the art, a dwell step may be performed prior to the surge step, if necessary. The process may be automatically repeated as desired until a sufficient quantity of platelets has been collected. The resultant product contains a high yield of platelets with reduced white blood cell contaminants.

### EXAMPLES

### Example 1

A single cavity centrifuge bowl having the construction shown in Fig. 1 was prepared. The outer diameter of the inner cavity 20 was about 50 mm and the inner diameter was about 33 mm. The maximum height of the cavity 20 was about 20 mm and the axial offset between the inlet and outlet peripheral slots 21 and 22 was about 10 mm. The volume of the cavity 20 was approximately 35 ml. The upper annular wall 24 was generally horizontal but the inclined wall 27 contained about 37 degrees with the axis of rotation.

The centrifuge bowl was mounted on a centrifuge machine for rotation, with the rotary seal assembly 30 fixedly supported. The bowl was initially filled with saline solution pumped at a predetermined flow rate and the bowl was started to rotate at a predetermined speed. After two minutes of feeding the saline, valves were operated to change the flow from saline to a fractionated whole blood containing platelets and white blood cells suspended in plasma. After switching the flow from saline to plasma, samples of eluted fluid were collected from the outlet port 32 at different points in time, and the collected samples were analyzed. The results are shown in Table 1. The number of platelets and white blood cells shown in Table 1 have been normalized to the volume of a standard platelet transfusion bag.

**Table 1**

| Flow Rate (ml/min) | Rotation (rpm) | Sample | Platelets Number (/200 ml) | Yield (%) | WBCs Number (/200 ml) |
|---|---|---|---|---|---|
| 80 | 4500 | fed plasma | 2.4 × 10¹¹ | - | 4.8 × 10⁷ |
| | | 1.0 min | 1.9 × 10¹¹ | 82.3 | 1.2 × 10⁵ |
| | | 1.5 min | 2.0 × 10¹¹ | 83.0 | 4.0 × 10⁴ |
| | | 2.0 min | 2.0 × 10¹¹ | 83.8 | 1.6 × 10⁵ |
| 80 | 3500 | fed plasma | 3.2 × 10¹¹ | - | 1.4 × 10⁸ |
| | | 1.0 min | 2.5 × 10¹¹ | 77.8 | 7.2 × 10⁵ |
| | | 1.5 min | 2.6 × 10¹¹ | 81.3 | 5.6 × 10⁵ |
| | | 2.0 min | 2.7 × 10¹¹ | 82.9 | 5.6 × 10⁵ |
| 100 | 4500 | fed plasma | 2.4 × 10¹¹ | - | 4.8 × 10⁷ |
| | | 0.8 min | 1.8 × 10¹¹ | 76.6 | 1.6 × 10⁴ |
| | | 1.2 min | 1.8 × 10¹¹ | 76.9 | 4.0 × 10⁴ |
| | | 1.6 min | 1.9 × 10¹¹ | 78.5 | 4.0 × 10⁴ |
| 100 | 4000 | fed plasma | 2.4 × 10¹¹ | - | 4.8 × 10⁷ |
| | | 0.8 min | 1.5 × 10¹¹ | 67.2 | 8.0 × 10⁴ |
| | | 1.2 min | 1.7 × 10¹¹ | 71.7 | <2.0 × 10⁴ |
| | | 1.6 min | 1.7 × 10¹¹ | 71.4 | 8.0 × 10⁴ |
| 100 | 3500 | fed plasma | 3.2 × 10¹¹ | - | 1.4 × 10⁸ |
| | | 0.8 min | 2.7 × 10¹¹ | 85.8 | 8.8 × 10⁵ |
| | | 1.2 min | 2.8 × 10¹¹ | 87.7 | 6.8 × 10⁵ |
| | | 1.6 min | 2.7 × 10¹¹ | 83.9 | 3.6 × 10⁵ |
| | | 2.0 min | 2.8 × 10¹¹ | 87.8 | 4.4 × 10⁵ |

From the results of Table 1, it is expected that the bowl of Fig. 1 has the ability to produce, from a platelet-rich-plasma fraction of whole blood, a 200 ml leukopoor product which may contain 2.0-3.0 × 10⁻¹¹ platelets, with less than 1 × 10⁶ white blood cell contaminant. Similarly, if the bowl of Fig. 4 is used to separate whole blood in the outer annular cavity 150 and intermediate density blood components enter the inner annular cavity 120 through the annular channel 121 for processing, platelets are harvested at a high yield with a lower level of contamination with white blood cells.

As has been described above, an improved and highly advantageous centrifuge bowl for processing particles, in particular blood cells such as platelets and white blood cells, is provided in accordance with the present invention. The terms and expressions employed herein are used as terms of description and not of limitation, and there is no intention, in the use of such terms and expressions, of excluding any equivalents of the features shown and described or portions thereof, but it is recognized that various modifications are possible within the scope of the invention claimed.

### Brief Description of Drawings

Fig. 1 is an axial sectional view of one embodiment of the centrifuge bowl comprising a single annular cavity in accordance with the present invention;
Fig. 2 is an axial sectional view explaining the expected function of the annular cavity of Fig. 1;
Fig. 3 is a partial cutaway elevational view illustrating the rotary seal assembly used for the centrifuge bowl of Fig. 1;
Fig. 4 is an axial sectional view of another embodiment of the centrifuge bowl comprising inner and outer annular cavities in accordance with the present invention; and
Fig. 5 is an explanatory view showing a dimensional relationship of the centrifuge bowl of Fig. 4 with the conventional Latham bowl.

## Claims

1. A centrifuge bowl comprising:
a bowl body adapted for rotation about a rotation axis;
an inlet port;
an outlet port;
at least one annular cavity formed in said bowl body, said cavity being concentrically located about said rotation axis and configured such that its annular cross sectional area taken parallel to said rotation axis increases from a centrifugal side of the cavity toward a centripetal side of the cavity;
a cavity inlet formed at said centrifugal side for communicating said cavity with said inlet port; and
a cavity outlet formed at said centripetal side for communicating said cavity with said outlet port.

2. The centrifuge bowl of claim 1, wherein said bowl body has an aperture at one axial end thereof and a rotary seal assembly is affixed to said bowl body to cover said aperture, said inlet and outlet ports are mounted to said rotary seal assembly.

3. The centrifuge bowl of claim 1, wherein said cavity inlet comprises a peripheral slot formed at said centrifugal side.

4. The centrifuge bowl of claim 1 wherein said cavity outlet comprises a peripheral slot formed at said centripetal side.

5. The centrifuge bowl of claim 1, wherein said cavity inlet and said cavity outlet are axially offset from each other.

6. The centrifuge bowl of claim 1, wherein said cavity terminates at said centripetal side with an annular wall extending substantially parallel to said rotation axis and having vertical upper and lower edges, and said cavity outlet is disposed at a position vertically intermediate the upper and lower edges of said annular wall.

7. The centrifuge bowl of claim 1, wherein said cavity is approximately triangular or quadrangular in axial cross section.

8. The centrifuge bowl of claim 1, wherein said cavity is asymmetric in axial cross section with respect to a line containing said cavity inlet and said cavity outlet.

9. The centrifuge bowl of claim 1, wherein an annular core is disposed in said bowl body for rotation therewith, and said cavity is formed between said bowl body and said core.

10. The centrifuge bowl of claim 9, wherein said bowl body is a two-part construction comprising an upper portion and a lower portion, said cavity is defined between said upper portion and said core, a radial passage is formed between said core and said lower portion for communicating said inlet port with said cavity inlet and a radial passage is formed between said core and said upper portion for communicating said outlet port with said cavity outlet.

11. A centrifuge bowl for processing a fluid containing particles, comprising:
a bowl body adapted for rotation about a rotation axis and having an aperture at one axial end thereof;
a rotary seal assembly affixed to said bowl body to cover said aperture and having inlet and outlet ports in fluid communication with the interior of said bowl body;
a core disposed within said bowl body for rotation therewith, said bowl body and said core being adapted to define:
at least one annular cavity for separating the particles, said cavity being concentrically located about said rotation axis and configured such that the velocity of the fluid entering the cavity decreases as the fluid moves from a centrifugal side of the cavity toward a centripetal side of the cavity;
a cavity inlet at said centrifugal side for fluidly communicating said cavity with said inlet port; and
a cavity outlet formed at said centripetal side for fluidly communicating said cavity with said outlet port.

12. The centrifuge bowl of claim 11, wherein said cavity inlet comprises a peripheral slot formed at said centrifugal side and said cavity outlet comprises a peripheral slot formed at said centripetal side, and wherein said peripheral slots are axially offset from each other.

13. The centrifuge bowl of claim 11, wherein said cavity terminates at said centripetal side with an annular wall extending substantially parallel to said rotation axis and having vertical upper and lower edges, and said cavity outlet is disposed at a position vertically intermediate the upper and lower edges of said annular wall.

14. The centrifuge bowl of claim 11, wherein said fluid contains particles of similar densities but of different diameters.

15. The centrifuge bowl of claim 11, wherein said fluid is a fractionated whole blood.

16. The centrifuge bowl of claim 15, wherein said fractionated whole blood comprises platelets and white blood cells suspended within plasma.

17. A centrifuge bowl for processing a fluid containing particles, comprising:
a bowl body adapted for rotation about a rotation axis and having an aperture at one axial end thereof;
a rotary seal assembly affixed to said bowl body to cover said aperture and having inlet and outlet ports in fluid communication with the interior of said bowl body;
a cavity for separating the particles, the cavity extending between a centrifugal side and a centripetal side;
a cavity inlet at said centrifugal side for fluidly communicating said cavity with said inlet port; and
a cavity outlet formed at said centripetal side thereof for fluidly communicating said cavity with said outlet port,
wherein said cavity is configured such that, upon rotation of the centrifuge bowl and with the fluid entering the cavity, an almost rigidly rotating flow filed is established in the cavity and Coriolis force is circumferantially dispersed substantially uniformly thereby.

18. The centrifuge bowl of claim 17, wherein said cavity is an annular cavity adapted to decrease the velocity of the fluid entering the cavity as the fluid migrates from said centrifugal side.

19. The centrifuge bowl of claim 17, wherein said cavity is an annular cavity, the annular cross sectional area of which, taken parallel to said rotation axis, increases from said centrifugal side toward said centripetal side.

20. The centrifuge bowl of claim 17, wherein said fluid contains particles of similar densities but of different diameters.

21. The centrifuge bowl of claim 17, wherein said fluid is a fractionated whole blood.

22. The centrifuge bowl of claim 21, wherein said fractionated whole blood comprises platelets and white blood cells suspended within plasma.

23. A centrifuge bowl comprising:
a bowl body adapted for rotation about a rotation axis;
an inlet port;
an outlet port;
an annular inner cavity formed in said bowl body, said inner cavity being concentrically located about said rotation axis;
an annular outer cavity formed in said bowl body, said outer cavity being concentrically located about said rotation axis radially outwardly of said inner cavity;
a cavity inlet formed at a centrifugal side of said outer cavity for communicating said outer cavity with said inlet port;
a cavity outlet formed at a centripetal side of said inner cavity for communicating said inner cavity with said outlet port; and
an annular restriction channel communicating said inner cavity with said outer cavity.

24. The centrifuge bowl of claim 23, wherein a core is disposed within said bowl body for rotation therewith, said bowl body and said core defining said outer and inner cavities and said annular restriction channel, said annular restriction channel radially communicates a centripetal periphery of said outer cavity with a centrifugal periphery of said inner cavity.

25. The centrifuge bowl of claim 23, wherein said bowl body has an aperture at one axial end thereof and a rotary seal assembly is affixed to said bowl body to cover said aperture, said inlet and outlet ports are mounted to said rotary seal assembly.

26. The centrifuge bowl of claim 23, wherein said cavity inlet comprises a peripheral slot formed at said centrifugal side of the outer cavity.

27. The centrifuge bowl of claim 23 wherein said cavity outlet comprises a peripheral slot formed at said centripetal side of the inner cavity.

28. The centrifuge bowl of claim 24, wherein said annular restriction channel and said cavity outlet are axially offset from each other.

29. The centrifuge bowl of claim 23, wherein said inner cavity terminates at said centripetal side with an annular wall extending substantially parallel to said rotation axis and having vertical upper and lower edges, and said cavity outlet is disposed at a position vertically intermediate the upper and lower edges of said annular wall.

30. The centrifuge bowl of claim 23, wherein said inner cavity being configured such that its annular cross sectional area taken parallel to said rotation axis increases from said annular restriction channel toward said centripetal side of the cavity.

31. The centrifuge bowl of claim 30, wherein said outer cavity being configured such that its annular cross sectional area taken parallel to said rotation axis decreases from said cavity inlet toward said annular restriction channel.

32. The centrifuge bowl of claim 30, wherein said inner cavity is approximately triangular or quadrangular in axial cross section.

33. The centrifuge bowl of claim 31, wherein said outer cavity is approximately rectangular in axial cross section.

34. The centrifuge bowl of claim 30, wherein said inner cavity is asymmetric in axial cross section with respect to a line containing said annular restriction channel and said cavity outlet.

35. The centrifuge bowl of claim 30, wherein said inner cavity is vertically bounded by upper and lower annular walls surrounding said rotation axis, said upper wall contains an acute angle with the rotation axis and said lower wall contains an obtuse angle with the rotation axis.

36. The centrifuge bowl of claim 24, wherein said bowl body is a two-part construction comprising an upper portion and a lower portion, said inner and outer cavities and said annular restriction channel is defined between said upper portion and said core, a radial passage is formed between said core and said lower portion for communicating said inlet port with said cavity inlet and a radial passage is formed between said core and said upper portion for communicating said outlet port with said cavity outlet.

37. A centrifuge bowl for processing a fluid containing particles, comprising:
a bowl body adapted for rotation about a rotation axis and having an aperture at one axial end thereof;
a rotary seal assembly affixed to said bowl body to cover said aperture and having inlet and outlet ports in fluid communication with the interior of said bowl body;
an annular outer cavity adapted for separating the particles according to density, said outer cavity being concentrically located about said rotation axis;
an annular inner cavity adapted for separating the particles by centrifugal elutriation, said inner cavity being concentrically located about said rotation axis radially inwardly of said outer cavity;
a cavity inlet formed at a centrifugal side of said outer cavity for communicating said outer cavity with said inlet port;
a cavity outlet formed at a centripetal side of said inner cavity for communicating said inner cavity with said outlet port; and
an annular restriction channel communicating said inner cavity with said outer cavity.

38. The centrifuge bowl of claim 37, wherein said inner cavity is configured such that the velocity of the fluid entering the inner cavity decreases as the fluid moves from the annular restriction channel toward the cavity outlet.

39. The centrifuge bowl of claim 37, wherein said cavity outlet comprises a peripheral slot formed at the centripetal side of said inner cavity, and wherein said peripheral slot is axially offset from said annular restriction channel.

40. The centrifuge bowl of claim 37, wherein said inner cavity is asymmetric in axial cross section with respect to a line containing said annular restriction channel and said cavity outlet.

41. The centrifuge bowl of claim 37, wherein a core is disposed within said bowl body for rotation therewith, said bowl body and said core defining said outer and inner cavities and said annular restriction channel, said annular restriction channel radially communicates a centripetal periphery of said outer cavity with a centrifugal periphery of said inner cavity.

42. The centrifuge bowl of claim 41 wherein said cavity outlet comprises a peripheral slot formed between said core and said bowl body at said centripetal side of the inner cavity.

43. The centrifuge bowl of claim 37, wherein said inner cavity terminates at said centripetal side with an annular wall extending substantially parallel to said rotation axis and having vertical upper and lower edges, and said cavity outlet is disposed at a position vertically intermediate the upper and lower edges of said annular wall.

44. The centrifuge bowl of claim 37, wherein said inner cavity being configured such that its annular cross sectional area taken parallel to said rotation axis increases from said annular restriction channel toward said centripetal side of the cavity.

45. The centrifuge bowl of claim 37, wherein said fluid contains particles of different densities and particles of similar densities but of different diameters.

46. The centrifuge bowl of claim 37, wherein said fluid is whole blood.

47. A centrifuge bowl for processing a fluid containing particles, comprising:
a bowl body adapted for rotation about a rotation axis and having an aperture at one axial end thereof;
a rotary seal assembly affixed to said bowl body to cover said aperture and having inlet and outlet ports in fluid communication with the interior of said bowl body;
a core disposed within said bowl body for rotation therewith, said bowl body and said core defining:
an annular outer cavity adapted for separating the particles according to density, said outer cavity being concentrically located about said rotation axis;
an annular inner cavity adapted for separating the particles by centrifugal elutriation, said inner cavity being concentrically located about said rotation axis radially inwardly of said outer cavity;
a cavity inlet at a centrifugal side of said outer cavity for communicating said outer cavity with said inlet port;
a cavity outlet at a centripetal side of said inner cavity for communicating said inner cavity with said outlet port; and
an annular restriction channel communicating said inner cavity with said outer cavity,
wherein said inner cavity is configured such that, upon rotation of the centrifuge bowl and with the fluid entering the inner cavity, an almost rigidly rotating flow filed is established in the inner cavity and Coriolis force is circumferantially dispersed substantially uniformly thereby.

48. The centrifuge bowl of claim 47, wherein said inner cavity is adapted to decrease the velocity of the fluid entering the inner cavity as the fluid migrates from said annular restriction channel.

49. The centrifuge bowl of claim 47, wherein said inner cavity has an annular cross sectional area taken parallel to said rotation axis, said annular cross sectional area increases from said annular restriction channel toward said centripetal side.

50. The centrifuge bowl of claim 47, wherein said fluid is whole blood, said outer cavity is adapted for separating the whole blood by centrifugation into red blood cells, buffy coat containing platelets and white blood cells and plasma, and said inner cavity is adapted for separating the platelets and white blood cells by centrifugal elutriation.
